# FASCICULE DE BREVET EUROPEEN

(11) **EP 1 159 427 B1**
(45) Date de publication et mention de la délivrance du brevet: **04.07.2007**
(21) Numéro de dépôt: 99973070.8
(22) Date de dépôt: 29.11.1999
(51) Int. Cl.: C07K 14/16, A61K 38/16

(54) **MUTANTS DE LA GP120 ET LEURS APPLICATIONS BIOLOGIQUES**
GP120-MUTANTEN UND DEREN BIOLOGISCHE VERWENDUNGEN
GP120 MUTANTS AND BIOLOGICAL APPLICATIONS

(30) Priorité: 27.11.1998 FR 9814997
(43) Date de publication de la demande: 05.12.2001
(73) Titulaire: Institut de Recherche pour le Développement ( IRD), 75480 Paris Cedex 10 (FR)
(72) Inventeur: VEAS, Francisco, F--34130 Mauguio (FR); CERUTTI, Martine, F-30380 Saint-Christol-lez-Alès (FR)
(74) Mandataire: Peaucelle, Chantal
(86) Numéro de dépôt international: PCT/FR1999/002949
(87) Numéro de publication internationale: WO 2000/032786

(56) Documents cités:
- US-A- 5 792 459
- QUIÑONES-MATEU, M.E. ET AL.: "Molecular characterizaton of human immunodeficiency virus type I isolates from Venezuela." AIDS RESEARCH AND HUMAN RETROVIRUSES, vol. 11, no. 5, 1995, pages 605-16, XP002119921
- MISSE D ET AL: "Dissociation of the CD4 and CXCR4 binding properties of human immunodeficiency virus type 1 gp120 by deletion of the first putative alpha-helical conserved structure" JOURNAL OF VIROLOGY, vol. 72, no. 9, 1 septembre 1998 (1998-09-01), pages 7280-7288, XP002086991 ISSN: 0022-538X cité dans la demande
- WILLEY, R.L. ET AL.: "Amino acid substitutions in the human immunodeficiency virus type 1 gp120 V3 loop that change viral tropism also alter physical and functional properties of the virion envelope." JOURNAL OF VIROLOGY, vol. 68, no. 7, juillet 1994 (1994-07), pages 4409-19, XP002119922
- THALI, M. ET AL.: "Discontinuous, conserved neutralization epitopes overlapping the CD4-binding region of human immunodeficiency virus type 1 gp120 envelope glycoprotein." JOURNAL OF VIROLOGY, vol. 66, no. 9, septembre 1992 (1992-09), pages 5635-41, XP002119923
- CORDONNIER, A. ET AL.: "Single amino-acid changes in HIV envelope affect viral tropism and receptor binding." NATURE, vol. 340, 17 août 1989 (1989-08-17), pages 571-4, XP002119924 cité dans la demande
- KORBER, B.T.M. ET AL.: "Genetic differences between blood- and brain-derived viral sequences from huamn immunodeficiency virus type 1-infected patients: evidience of conserved elements in the V3 region of the envelope protein of brain-derived sequences." JOURNAL OF VIROLOGY, vol. 68, no. 11, novembre 1994 (1994-11), pages 7467-81, XP002119927
- LUND, O. ET AL.: "Inhibition of HIV type 1 infectivity by coexpression of a wild-type and a defective glycoprotein 120." AIDS RESEARCH AND HUMAN RETROVIRUSES, vol. 14, no. 16, 1 novembre 1998 (1998-11-01), pages 1445-50, XP002119925
- KWONG, P.D. ET AL.: "Structure of an HIV gp120 envelope glycoprotein in complex with the CD4 receptor and a neutralizing human antibody." NATURE, vol. 393, 18 juin 1998 (1998-06-18), pages 648-59, XP002119926 cité dans la demande

## Description

L'invention vise des mutants de la gp120 et leurs applications.

On sait que les interactions entre le CD4 de cellules cibles et la gp120 de VTH impliquent des régions conservées de la gp120, en particulier un tryptophane (W) en position 427 selon la numérotation des acides aminés de Kwong et al (réf 1. dans la liste bibliographique en fin de description).

Dans l'article de Missé et al (2), dont certains des coauteurs sont co-inventeurs de la présente demande, il a été démontré qu'une structure stable, dite conservée de la gp120, contenue dans la zone C1, à savoir l'hélice α1 amphipatique, est impliquée dans les interactions avec le récepteur CD4. De plus la construction d'une gp120 sans l'hélice α-1 a permis de démontrer qu'une interaction est toujours possible avec la cellule cible, en particulier avec le récepteur CXCR4, récepteur aux chimiokines sur les lymphocytes.

En induisant des mutations ponctuelles spécifiques dans les structures conservées de la gp120, et tout particulièrement de l'hélice α2, les inventeurs ont mis en évidence le rôle essentiel joué par ces structures dans les interactions avec les cellules cibles, que ce soit directement ou indirectement. En effet, la trans-conformation de ces structures du fait d'une mutation donnée a permis, comme illustré dans les exemples, de démasquer des régions normalement cachées qui sont impliquées dans ces interactions, et de disposer ainsi de molécules capables de servir de cibles vaccinales.

L'invention vise donc des mutants de la gp120, caractérisés en ce qu'ils comportent une mutation dans l'hélice α2 de la gp120, d'un motif tryptophane en position 338 en un motif sérine.

Cette mutation est située dans la région de la gp120 correspondant à la cavité d'interaction avec le CD4.

Cette mutation unique est capable d'induire une absence totale de fusion cellulaire lorsqu'on met en présence des cellules cibles CD4⁺, CXCR4' et des cellules qui expriment la gp120 mutée W338S.

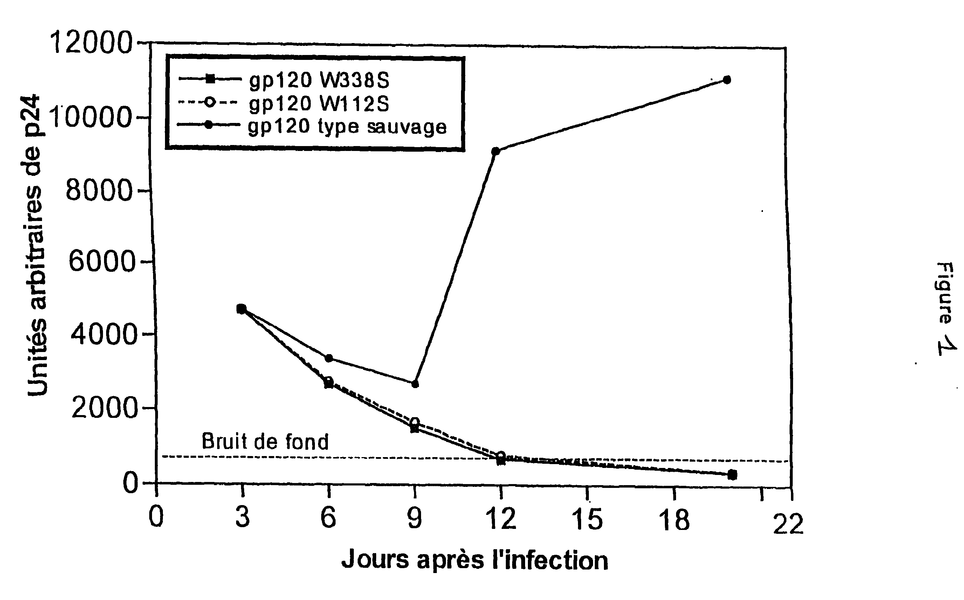

Le virus complémenté par une enveloppe comportant la mutation W338S au niveau de la gp120 n'est plus capable d'infecter les cellules (voir figure 1).

Comme noté plus haut, W faisant partie de la structure α2 conservée, cet acide aminé se trouve donc dans la deuxième poche contenant des résidus aromatiques et des résidus hydrophobes.

Cette mutation suggère alors que cette structure est impliquée dans l'interaction avec les co-récepteurs, par exemple CXCR4, compte tenu des résultats biologiques.

L'invention vise plus spécialement les mutants de la gp120 comportant outre la mutation W338S, une mutation dans l'hélice α1 de la gp120 des motifs tryptophane en positions 96 et 112 en un motif sérine.

Une telle mutation induit une absence totale de fusion cellulaire. Les tests de fusion cellulaire, qui sont rapportés ci-après dans la partie exemple, montrent ainsi que la substitution W112S abolit la formation de *syncytia* entre des cellules HeLa-Tat (exprimant une gp160 contenant cette mutation) et des cellules HeLa P4. De plus, un virus contenant cette même mutation est incapable d'infecter des lymphocytes humains. Une substitution de ce même W (résidu aromatique bicycle) par un résidu aromatique (monocyclique) diminue ces fonctions mais ne les supprime pas.

De plus, chez de tels mutants, la reconnaissance par des anticorps spécifiques du site de liaison à CD4 défini par Cordonnier et al (3) est fortement diminuée.

Ces résultats montrent donc le rôle capital de la structure conservée de l'hélice α1 pour la fixation au CD4.

En plus de la mutation en position 112, de tels mutants peuvent également comporter une mutation de F en position 383 en alanine et, le cas échéant, du tryptophane en position 427 remplacé par une glycine et/ou du tryptophane en position 479 remplacé par un acide aminé non aromatique, à savoir la sérine.

Les données cristallographiques publiées dans (1) ont permis de visualiser d'une part la position de l'hélice alpha 1 de la gp120 dans cette structure tridimensionnelle et d'autre part des résidus tryptophane.

Ces données ont confirmé les résultats des expériences biologiques évoquées ci-dessus.

En particulier, le W 112 se trouve dans la poche hydrophobe décrite comme poche d'interaction de la gp120 au CD4 ; cette poche est constituée d'un "cluster" de résidus aromatiques, qui interagirait avec un autre résidu aromatique la.F43 du CD4. En outre, il ressort de cette étude que les résidus tryptophane ont une position stratégique dans cette poche. Ainsi toute mutation de l'un d'entre eux déstabiliserait cette "cavité" et empêcherait toute liaison avec la F43 du CD4.

Les mutants selon l'invention, en permettant d'étudier le rôle des structures conservées constituent une nouvelle façon d'aborder les interactions agents infectieux-cellules cibles. Le fait de mieux appréhender la fonctionnalité des différentes structures conservées impliquées dans les interactions agents pathogènes-cellules cibles permet en effet la conception de molécules thérapeutiques ou immunisantes contre le VIH.

L'invention, mettant à profit la mise en évidence de ces poches d'interaction, vise des composés capables de mimer le CD4, et dotés ainsi de propriétés inhibitrices vis-à vis de la gp120.

Elle vise également des peptides mimant les boucles extracellulaires des co-récepteurs, capables de se loger dans lesdites poches hydrophobes.

L'invention concerne également des peptides capables de mimer les régions constantes de la gp120 évoquées ci-dessus, et susceptibles de constituer des candidats comme vaccins. L'invention fournit ainsi des modèles pour l'élaboration de tels candidats.

En outre, en effectuant des mutations sur la première cavité, il est possible d'induire des transformations de la gp120 permettant une meilleure exposition de la deuxième cavité, et par là de la gp120 en tant qu'antigène. De manière avantageuse, on obtient alors une réponse immune adaptée contre ces régions constantes habituellement cachées.

Par de telles transformations, il est également possible d'induire des dissociations entre la gp120 et la gp41. Comme montré dans les exemples donnés ci-après, on n'observe aucune fusion lorsqu'on utilise des virus complémentés par une gp160 comportant une mutation au niveau de la qp120, alors que la glycoprotéine responsable de la fusion est la gp41. La mutation sur la gp120 constitue donc une cible pour empêcher la fusion du virus. On dispose ainsi d'épitopes clés pour éviter l'infection par H1V.

D'autres caractéristiques et avantages de l'invention sont donnés dans les exemples qui suivent, dans lequels il et fait référence aux figures 1 à 8, qui représentent, respectivement,
- la figure 1, les résultats de tests d'infectivité de pseudovirus HIV-1 complémentés par une enveloppe comportant des mutations selon l'invention au niveau de la gp120,
- la figure 2, la structure cristalline de la gp120,
- la figure 3, les résultats de test d'activité biologique de rgp120, avec un anticorps polyclonal de lapin anti-gp120,
- la figure 4, la réactivité de rgp120 avec l'AcM CG10,
- la figure 5, la réactivité de rgp120 complexées au CD4 avec l'AcM CG10,
- La figure 6, la réactivité de rgp120 avec l'AcM 4.8d,
- la figure 7, la réactivité de rgp120 complexées au CD4 avec l'AcM 4.8d, et
- la figure 8, la réactivité de rgp120 avec l'AcM 17b.

### Exemple : obtention et étude de mutants de la gp120.

Ces mutants de gp120 ont été testés dans plusieurs systèmes biologiques :
(i) la fusion cellulaire (enveloppes mutées à la surface des cellules), (ii) les infections virales (pseudo-virus complémentés par des gp1.20 mutées), (iii) l'analyse de la topologie de ces gp120 mutées faite par différents anticorps monoclonaux reconnaissant différents épitopes de la gp120 dans le "CD4 binding site", et (iv) l'analyse de la gp120 à travers les données cristallographiques.

Ces expériences ont été réalisées en utilisant les matériels méthodes suivants :

### . Plasmides, anticorps et pseudovirus

Le plasmide pHXB2ENV (code pour l'enveloppe gp160 du VIH-IIIE), le plasmide pCMV-rev (code pour la protéine Rev sous le contrôle du promoteur CMV), et l'anticorps monoclonal 902 (dirigé contre la boucle V3 de la gp120), onL été obtenus du NIH AIDS Research and Référence Reagent Program (EU). Le pseudovirus pNL4-3env-GFP a été fourni par le Dana-Farber Cancer Institute, Boston, EU. Ce plasmide contient le génome du VIH-1 NL4-3 délété du gène Env, ainsi qu'une substitution du gène Nef par le gène codant pour la protéine GFP (Green Fluorescent Protein).

### . Mutagénèse dirigée dans l'hélice alpha 1 de la gp120

Dans le but d'étudier les résidus W présents dans la structure hélice alpha 1 de la gp120, la région NdeI-HincII du gène codant pour la gp120 a été reconstitué. A cet effet on a utilisé un oligonucléotide dans lequel ont été introduits deux sites de restriction (HpaI et HindIII) entourant l'hélice α 1 (2). L'hélice α 1 de la gp120 possède deux résidus tryptophane (W96 et W112) (4). Les sites HpaI et Hind III ont permis d'échanger pour chaque construction un fragment HPaI-Hind III sauvage par un fragment HpaI-Hind III muté. Les mutations ont été effectuées soit simultanément sur les deux W, soit sur un seul tryptophane. La mutagénèse dirigée a été effectuée par la méthode des oligonucléotides chevauchants compris entre le site Hpal et Hind III du plasmide pBS. Dans le tableau 1 ci-dessous ne figurent que les oligonucléotides qui comportent la mutation souhaitée.

**Tableau 1**

| **Mutants** | **oligonucléotides** |
|---|---|
| 96W/S | ⁵ AAC GTC ACA GAA AAT TTT AAC ATG AGT AAA AAT G³ |
| 112W/S | ⁵ GAT ATA ATC AGT TTA TCT GAT CAA AGC³ |
| 96W | ⁵ AAC GTG ACA GAA AAT TTT AAC ATG TGG AAA AAT G³ |
| 112W | ⁵ GAT ATA ATC AGT TTA TGG GAT CAA AGC³ |

### . Fusion cellule-cellule

Les vecteurs d'expression contenant les différentes enveloppes gp160 (pHXB2ENV, 500ng) ont été co-transfectés avec 500 ng de pCMV-rev dans des cellules HeLa-Tat. La méthode de transfection utilisée a été la lipofection (lipofectamine, GIBCO) comme décrit dans (2).

Le pCMV-rev est indispensable pour activer l'expression du gène ENV présent dans le plasmide pHXB2 ENV. Les cellules transfectées ont ensuite été mises en coculture avec les cellules HeLaP4 (CD4-LTR LacZ) comme décrit sans (2).

### . Expression à la surface cellulaire des gp120 mutées

La technique de transfection utilisée a été la même que celle employée dans le cas de la fusion cellule-cellule.

Vingt-quatre heures avant la co-transfection, les cellules HeLa-Tat ont été placées dans des puits de 3,5 cm de diamètre recouverts de lamelles de verre (12 mm de diamètre). Les cellules ont été incubées 48 heures après la co-transfection avec l'Acm 902 (dirigé contre la boucle V3 de la gp120) à la concentration de 10 µg/ml. Cette incubation a été réalisée en présence de 0,1 % d'azide de sodium (NaN₃) et en tampon PBS. Après 2 lavages en PBS 0,3 % BSA /0,1 NaN₃, les cellules ont ensuite été incubées avec un anticorps anti-IgG de souris marqué au Texas-red (Molecular probes). Après 3 lavages extensifs, les cellules ont été fixées pendant 2 minutes avec un mélange éthanol-acétone (1:2). Après montage des lamelles sur des lames avec du Mowiol, les cellules ont été observées en microscopie confocale.

### . Dosage de la p24 par ELFA (Enzyme linked fluorescent assay)

Le dosage de la p24 est effectué avec le système automatisé VIDAS VIH DUO (BioMérieux). Ce test repose sur une détection simultanée de l'antigénémie p24 et des anticorps anti-VIH-1 et anti-VIH-2. Le principe du test associe deux réactions immuno-enzymatiques à une détection en fluorescence. Ce test est muni d'un cône à usage unique qui sert à la fois de phase solide et de système de pipetage. Dans la partie basse du cône, sont fixés trois peptides de synthèse (gp41, gp36 et un peptide spécifique du type O) conjugués à des Acms de souris anti-IgG humaines marqués à la phosphatase alcaline. Dans la partie haute du cône, sont fixés des Acms anti-p24. Lors de la réalisation du test, un anticorps de lapin anti-p24 biotinylé (conjugué à la streptavidine-phosphatase alcaline) est ajouté en même temps que l'échantillon. Le substrat, 4-méthyl-ombelliferyl-phosphate, permet de mesurer la fluorescence qui est proportionnelle à la quantité d'Ac anti-VIH et/ou d'Ag p24 présents dans l'échantillon. La réaction est réalisée en 90 minutes et une lecture de la fluorescence est faite avec le lecteur spécifique du test VIDAS (longueur d'onde de 450 nm).

### . Infections des lymphocytes humains par des pseudo-virus mutés obtenus par complémentation.

Des cellules 293 ont été co-transfectées avec 10 µg de pseudovirus pNL4-3 GFP, 2 µg du plasmide pHXB2 ENV (contenant ou non les différentes mutations dans la SU gp120) et 1,5 µg du plasmide pCMV-rev. Pour cela, la méthode de transfection au phosphate de calcium a été utilisée. Quarante-huit heures après la transfection, les surnageants des cellules ont été prélevés, filtrés et conservés à -80°C jusqu'à l'utilisation. Une mesure de la p24 a ensuite été effectuée sur ces différents surnageants. Des lymphocytes humains stimulés prélablement pendant 3 jours par 10 µg/ml de PHA (Phytohémaglutinine), 50 U d'IL-2 dans un milieu de culture RPMI 1640-10 % SVF (Sérum de Veau Foetal), ont été incubés avec les différents surnageants de cellules 293 transfectées (contenant une concentration identique de p24).

Les capacités infectieuses des différents surnageants ont été visualisées par microscopie à fluorescence (expression de la protéine GFP dans les lymphocytes) et déterminées par un dosage de la p24 (suivi périodique tous les 3 jours) dans les surnageants de culture de lymphocytes infectés.

### . Analyses Structurales

La structure du complexe constituée par le coeur de la gp120, les deux premiers domaines du CD4 et le fragment Fab de l'anticorps neutralisant 17b a été récemment déterminée par cristallographie aux rayons X (1). Cette structure a été déposée dans protéine data base (pdb) sous le code d'accès "Igcl". Les coordonnées structurales de ce complexe ont été analysées avec le logiciel Insight I (MSI pour Molecular Simulations) sur la station graphique Oxygène (Silicon grafic O₂). La visualisation et la modélisation des domaines de la gp120 et du CD4 ont été effectués à l'aide du programme Discover (MSI) sous l'environnnement Insigth II.

### . Résultats

### . Capacités fusiogènes des enveloppes gp160

Afin d'évaluer les capacités fusiogènes des différentes enveloppes gp160 exprimées à la surface des cellules HeLa-Tat transfectées, ces cellules ont été mises en co-culture avec des cellules HeLa P4 (CD4 LTR LacZ).

Les résultats obtenus sont donnés dans le tableau 2 suivant

| Mutations dans la gp120 | Pourcentage de foyers de fusion |
|---|---|
| Type sauvage | 100% (environ 1500 foyers de fusion/puits) |
| 96W/S et 112W/S | 0% |
| 96W/I et 112W/I | 0% |
| 96W/S | Non déterminé |
| 112W/S | 0% |
| 427W/S | 0% |
| 338W/S | 0% |
| Contrôle | 0% |

On constate à l'examen de ces résultats que des mutations simultanées sur les deux résidus tryptophane de l'hélice alpha 1 (W96 et W112) en sérine rendent l'enveloppe gp 160 incapable d'induire des *syncytia* entre les cellules. Le même phénomène est observé avec l'enveloppe gp 160 contenant la mutation ponctuelle 112 W/S et/ou 338 W/S. En revanche, la substitution d'un résidu tryptophane (W) par un résidu aromatique phénylalanine (F), d'indice hydrophobicité proche, réduit d'environ 50 % les capacités fusiogènes des enveloppes mutées. On peut constater que, quelle que soit la construction, ce phénomène est observé uniquement lorsque le résidu W112 et/ou le résidu W338 est (sont) impliqué(s), ce qui dénote l'importance biologique de ce ou ces résidus, par rapport au groupement W96 (dans le cadre de ces expériences). Tous ces résultats ont été comparés avec ceux obtenus avec l'enveloppe gp160 sauvage (dans laquelle on observe environ 1500 foyers de fusion). Le contrôle négatif était la mise en co-culture de cellules HeLa-Tat non transfectées avec des cellules HeLa P4 (aucun foyer de fusion n'a été observé).

Ces résultats indiquent que le résidu W112 et/ou le résidu W338 est (sont) impliqué(s) dans les interactions qp120-cellules cibles, qui précédent le phénomène de fusion cellulaire.

### . Expression à la surface cellulaire des protéines gp120

La gp120 a été détectée par l'Acm 902, dirigé contre la boucle V3 de la gp120. Ce dernier a ensuite été révélé par un anti-IgG de souris couplé au Texas red. On constate qu'aussi bien la gp120 sauvage que la gp120 112W/S, sont exprimées à la surface des cellules. L'incubation de l'anticorps 902 avec des cellules HeLa-Tat non transfectées ne donne aucun signal.

### Capacités infectieuses des peudo-virus complémentés

La figure 1 montre l'évolution du taux de p24 dans les surnageants de lymphocytes (préalablement activées par 10 µg/ml de PHA et 50 U / ml d'IL2) mis en contact avec :
- le pseudovirus complémenté par la gp120 de type sauvage (courbe - ● -)
- le pseudovirus complémenté par la gp120 mutée (gp120 112W/S) (- ○ -)
- le pseudovirus complémenté par la gp120 mutée (gp120 338W/S).(- ■ -)

Dans le premier cas, le taux de p24 diminue d'abord pour augmenter considérablement (à partir du 10ème jour après l'infection), ce qui prouve la présence d'une p24 synthétisée *de novo,* produit d'une infection virale.

Dans le deuxième cas, le taux de p24 diminue progressivement pour disparaître complètement au 20ème jour (après la mise en contact). Il n'y a donc pas eu infection des cellules grâce à la mutation 112 W/S dans la gp120. Ce fait a été confirmé par l'absence d'expression de la protéine GFP dans les cellules incubées avec le virus muté 112 W/S et/ou 338 W/S (visualisation en microscopie à fluorescence) .

L'absence d'infection des cellules observée dans ce dernier cas, peut-être expliqué par l'absence de formation de *syncytia* visualisée précédemment avec des cellules exprimant à leur surface la gp120 112 W/S et/ou la gp120 338 W/S .

### . Analyse tridimensionnelle et localisation structurale des mutations effectuées.

La structure cristallographique du complexe gp120-CD4-17b Fab a récemment été déterminée (3) avec une résolution de 2,5 Å (Figure 2).

Les coordonnées de cette structure ont été analysées à l'aide du logiciel Insight II, permettant de situer les acides aminés dans un contexte tridimensionnel beaucoup plus résolutif que les modèles prédictifs qui ont été à l'origine de notre démarche.

L'analyse de la structure cristallographique du complexe qp120-CD4 met en évidence un nombre de résidus réduits formant l'interface entre les deux protéines. L'interaction majoritaire entre la gp120 et le CD4 est localisée au niveau d'une poche hydrophobe, impliquant l'acide aminé W427 de la gp120 et le résidu F43 du CD4 (Wyatt et Coll., 1998).

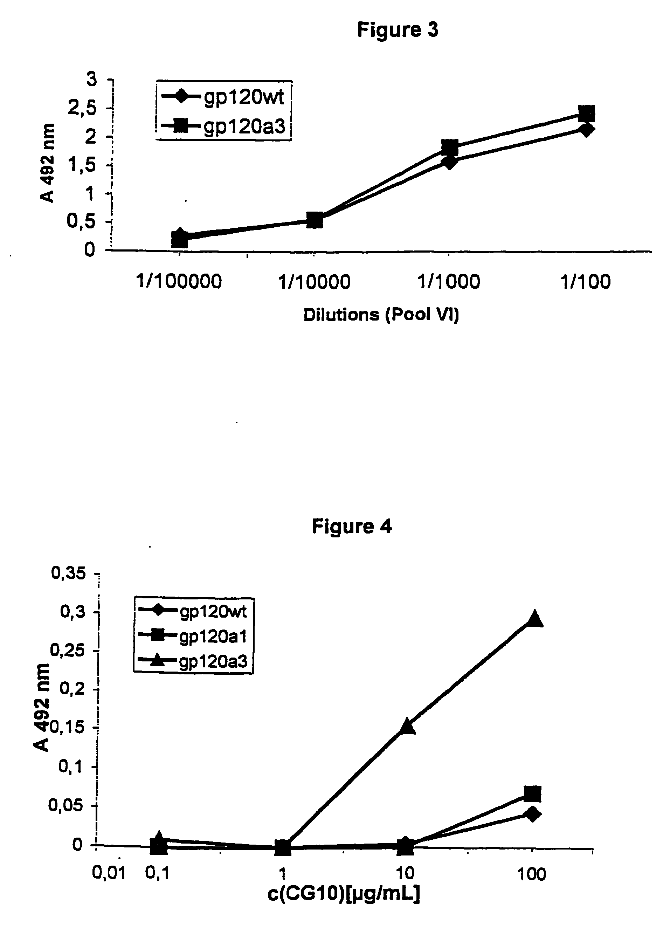

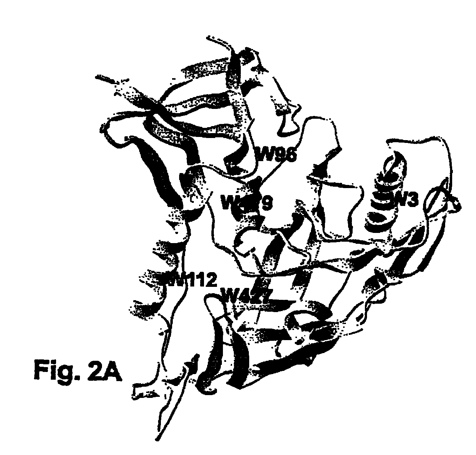

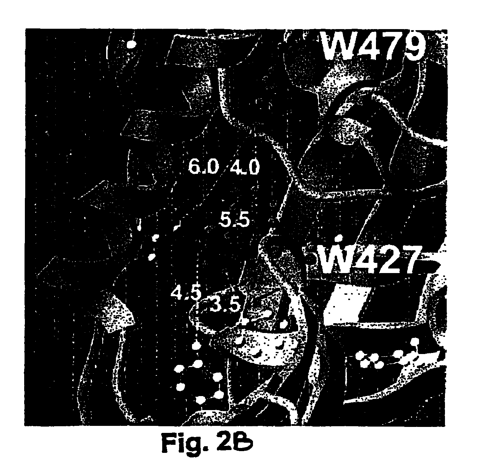

De plus, comme le montre la Figure 3, le W112 est stratégiquement localisé dans cette cavité riche en résidus aromatiques et interagit directement avec la F43 du CD4. Le résidu F43 se logerait au milieu de cette poche et interagirait directement avec W112. L'interaction du N112 avec la F43 et le W427 permettrait de stabiliser l'ensemble de la poche hydrophobe. On peut noter que le W112 et le W427 sont situés à une distance inférieure ou égale à 5 Å l'un de l'autre, ce qui nous indique que ces deux résidus tryptophane sont susceptibles d'interagir fortement.

Cette interaction pourrait être réalisée par effet d'empilement des noyaux aromatiques entre ces deux résidus ou par liaison dipôle-dipôle. De plus, ces distances peuvent s'avérer en réalité être beaucoup plus réduites en tenant compte de l'aspect dynamique des interactions moléculaire *in vivo.*

L'analyse cristalline a permis en outre de découvrir que W 338 de l'hélice α2 est susceptible de jouer un rôle majeur dans la stabilité de la deuxième cavité (sur un total de 2 cavités incluses à l'intérieur de la gp120), constituée des résidus hydrophobes et aromatiques. Dans ces conditions, cette cavité peut être considérée comme capitale dans les interactions avec la gp120 et les co-récepteurs, par exemple CXCR4.

### STRUCTURE:

### Etude ELISA : Fixation de la gp120 seulement sur le CD4 soluble

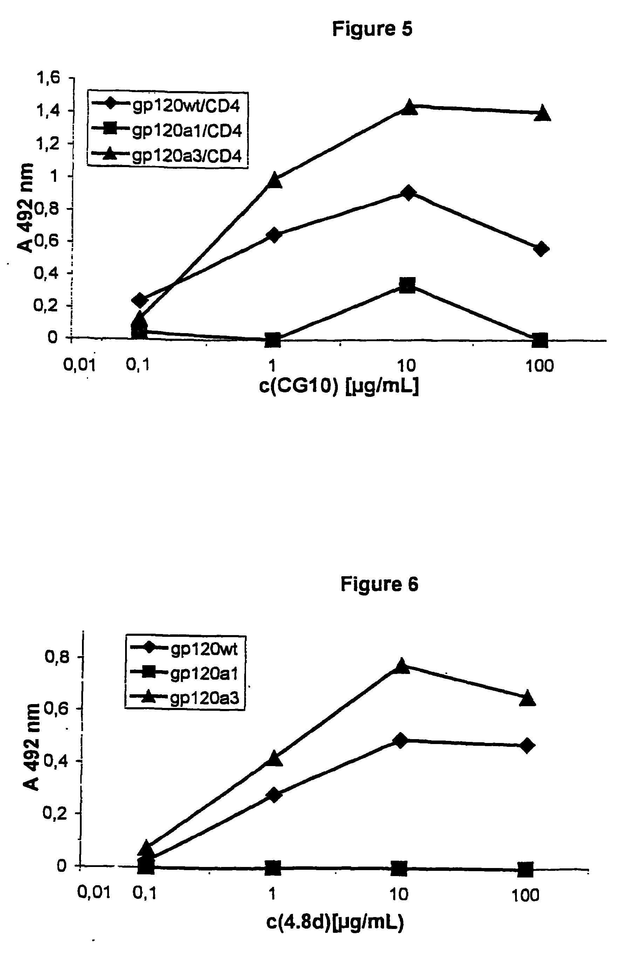

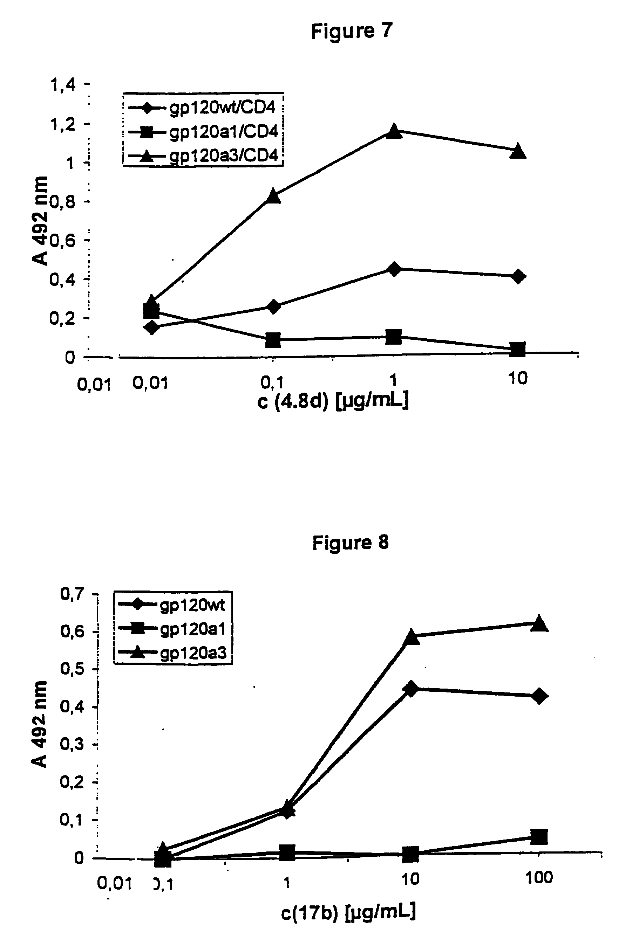

On rapporte sur les figures 4 à 8 les résultats de la réactivité des rgp120 sauvage gp120ΔαHX1 et gp120α3 W338S avec l'AcM CG10 (figure 4), l'AcM 4.8d (figure 6) et l'AcM 17b (figure 8) : dans ces essais, les rgp120 ont été déposés à la concentration de 1 µg/ml. L'AcM a été déposé aux concentrations indiquées en abscisse. Les figures 5 et 7 donnent les réactivités de ces rgp120 complexées au CD4 avec l'AcM CG10 (figure 5) et l'AcM 4.8d (figure 7) : dans ces essais, les rgp120 ont été déposées à la concentration de 1 µg/ml. La concentration de CD4 est de 20 µg/ml et l'AcM a été déposé aux concentrations indiquées en abscisse.

Cette mutation induit une trans-conformation qui permet une meilleure exposition aux anticorps monoclonaux 17b, 4.8d et G10 dirigés contre les épitopes CD4i (CD4 induit) (Fig. 4, 5, 6, 7 et 8) qui sont des épitopes démasqués après la fixation de la gp120 sur le CD4. Cette trans-conformation a pu être détectée dans un système ELISA qui a été effectué comme suit :
1. Le fond des puits est revêtu avec du CD4 soluble (référence NIH) ou avec l'anticorps Aalto D7324 (qui est un anticorps de chèvre) dirigé contre un peptide linéaire de la Région C-terminale de la gp120 (cet anticorps n'induit pas la trans-conformation pour obtenir le démasquage des épitopes CD4i) .
2. Après saturation avec la BSA 3 %, la gp120 est incubée avec le CD4 ou avec l'anticorps D7324 à 37°C pendant 1hr.
3. Suite à 2 lavages, l'un des AcM anti-CD41 (17b ou 4.8d ou CG10) est incubé avec la gp120 préalablement accrochée (type sauvage ou mutante) pendant 1hr a 37°C.
4. Les anticorps CD4i sont révélés avec un anticorps anti-IgG conjugué à la peroxydase (POD)
5. L'intensité de la réaction est lue dans le lecteur ELISA après arrêt de la réaction.

Le bruit de fond représenté par les contrôles (cellules sans gp120) es retiré des résultats de points expérimentaux.

Remarque : un contrôle a été fait avec des gp120 détectées par un anticorps polyclonal de lapin dirigé contre la gp120 (fig.3).

Ces expériences faites en ELISA ont permis de vérifier qu'en effet, les AcM anti-CD4i 17b et 4.8d reconnaissent mieux la gp120 sauvage et la gp120 mutée lorsqu'elle est associée au CD4 soluble, permettant le démasquage de ces épitopes. Alors que l'AcM CG10 ne reconnaît pas du tout la gp120 sauvage non-complexée au CD4, cet AcM reconnaît la gp120 mutée sans qu'elle soit complexée au CD4.

### Interprétation :

La mutation W 338S sur la gp120 induit le démasquage des épitopes CD4i, en particulier les épitopes reconnus par l'ACM CG10.

Ce résultat est important, car cette mutation fait de ce mutant un bon candidat vaccinal exposant des épitopes masqués chez les virus infectieux.

### Etude FACS : Fixation simultanée des gp120 sur au moins 2 récepteurs présents à la surface des cellules de la lignée lymphocytaires CEM, le CD4 et le CXCR4.

Afin de vérifier que les gp120 se fixent sur le CD4 et le CXCR4 respectivement l'expérience suivante a été réalisée : les cellules CEM sont d'abord incubées avec les gp120, puis incubées avec des anticorps monoclonaux contre le récepteur CD4 (AcM OKT4a FITC de chez ORTHO Diagnostic) et contre le récpteur CXCR4 (AcM 12G5 de chez Pharmingen) et ensuite avec les anticorps appropriés anti-IgG de souris.

On rapporte dans le tableau 3 la moyenne de l'intensité de fluorescence de gp120 fixée sur des cellules CEM CD4+/CXCR4+ et reconnues par des AcM anti-CD4.

**TABLEAU 3**

| Anticorps AcM anti-CD4i | INTENSITE MOYENNE DE FLUORESCENCE DE GP1:cr FIXEES AUX CELLULES CEM CD4+ /CXCR4+ | | |
|---|---|---|---|
| | sans gp120 | gp120 sauvage | gp120 W338S |
| 4.8d | 5,65 | 76,775 | 208, 78 |
| 17b | 5,21 | 70,48 | 175,575 |
| CG10 | 5,14 | 7,15 | 8,49 |

Aucun marquage n'a pu être observé, ce qui signifie que la gp120 est effectivement fixée sur le CD4 et sur le CXCR4.

Les cellules CEM cultivées dans RPMI 10% SVF, ont été lavées avec un tampon de lavage PBS/0,3%/BSA/0,02%Na-azide (TL) et mis dans un tampon d'incubation FBS/3%/BSA/0,02%Na-azide (TI) afin de les mettre en contact avec les gp120 sauvage et mutante pendant 1. heure à 37°C.

Les cellules ont été lavées 2 fois avec du TL, puis incubées dans du TI avec l'un des anticorps anti-CD41 (le 17b, le 4.8d ou le CG10) concentré à 10 µg/ml pendant 1 heure à 25°C. Les cellules ont été ensuite lavées 2 fois avec du TI., puis incubées pendant 45 minutes à 25°C dans du TI avec un anticorps anti-IgG approprié conjugué au FITC ou à la PE. Après 3 lavages avec du TL, les cellules sont remises en suspension dans du TI et passées au cytofluorimètre de flux (FACSort). 10 000 cellules sont ainsi analysées une à une.

Les résultats sont reportés sur la figure 5. L'anticorps 17b et 4.8d reconnaissent bien la gp120 mutée et très peu la gp120 sauvage. En revanche, l'AcM CG10 n'a plus accès à son site de reconnaissance ni sur la gp120 sauvage, ni sur la gp120 mutée.

### Interprétation

Si l'AcM CG10 n'a plus accès à son site et qu'en revanche les AcM 17b et 4.8d ont accès à ce site, ceci signifie que l'épitope de la gp120 impliqué fortement dans la fixation de la gp120 au co-récepteur CXCR4 est surtout celui qui est reconnu par le CG10.

### Conclusion

La mutation W338S sur la gp120 induit une trans-conformation qui empêche la fusion et l'infection. En effet, cette mutation empêche la fusion, lorsque l'enveloppe est exprimée à la surface de cellules transfectées et empêche l'infection lorsque cette enveloppe est située sur le virus complémenté. De plus, cette trans-conformation permet un démasquage de sites masqués sur la gp120 sauvage du fait, essentiellement, que la gp120 mutée est reconnue par l'AcM CG10 sans que cette gp120 soit complexée au CD4. Le fait que ce même AcM CG10 n'ait plus accès à son épitope lorsque cette protéine est associée au CD4 et au CXCR4, et que les anticorps .anti-CD4i n'interfèrent pas avec le CD4, implique que c'est l'épitope reconnu par l'AcM CG10 sur la gp120 qui est impliqué dans la fixation au CXCR4. Un te] mutant constitue un candidat pour effectuer des immunisations à des fins de protection contre l'infection VIH.

### REFERENCES BIBLIOGRAPHIQUES

1. Kwong PD., Wyatt R., Robinson J., Sweet R W., Sodroski J. and Hendrickson W A. 1998. Structure of an VIH gp120 enveloppe glycoprotein in complex with the CD4 receptor and neutralizing antibody. Nature. 393:648.
2. Missé D., Cerruti M., Schmidt I., Jansen A., Devauchelle G., Jansen F. and Véas F. 1998. Dissociation of the CD4 and CXCR4 binding properties of human immunodeficiency virus type 1 gp120 by deletion of the first putative alpha-helical conserved structure. J-Virol 72:7280.
3. Cordonnier A., Montagnier L., and Emmerman M. 1989. Single amino-acid changes in VIH envelope affect viral tropism and receptor binding. Nature 340:571.
4. Hansen J E., Lund O., Nielsen JO., Brunak S, and Hansen JES. 1996. Prédiction of the secondary structure of HIV-1 gp 120. Proteins 25:1.

## Revendications

1. Mutants de la gp120, **caractérisés en ce qu'**ils comportent une mutation dans l'hélice α2 de la gp120 d'un motif tryptophane en position 338 en un motif sérine.

2. Mutants selon la revendication 1. **caractérisés en ce qu'**ils comportent une mutation dans l'hélice α1 de la gp120 des motifs tryptophane en positions 96 et 112 en un motif sérine.

3. Mutants selon la revendication 1 ou 2, **caractérisés en ce qu'**ils comportent en outre une mutation de F en position 383 en alanine, et le cas échéant une mutation du trytophane en position 427 en glycine, et/ou du tryptophane en position 479 en sérine.

4. Application des mutants selon l'une quelconque des revendications 1 à 3, en tant qu'antigènes pour l'élaboration de vaccins.

## Claims

1. Mutants of gp120, **characterized in that** they comprise a mutation in the α2 helix of gp120 of a tryptophan moiety in position 338 to a serine moiety.

2. Mutants according to claim 1, **characterized in that** they comprise a mutation in the α1 helix of gp120 of the tryptophan moieties in positions 96 and 112 to a serine moiety.

3. Mutants according to claim 1 or 2, **characterized in that** they also comprise an mutation of F in position 383 to alanine and if appropriate a mutation of tryptophan in position 427 to glycine and/or of tryptophan in position 479 to serine.

4. Use of mutants according to any one of claims 1 to 3, as antigens for the production of vaccines.

## Patentansprüche

1. Mutanten von gp120, **dadurch gekennzeichnet, dass** sie in der Helix α2 von gp120 eine Mutation eines Tryptophanrests an Position 338 zu einem Serinrest tragen.

2. Mutanten gemäß Anspruch 1, **dadurch gekennzeichnet, dass** sie eine Mutation in der Helix α1 von gp120 von Tryptophanresten an den Positionen 96 und 112 zu Serinresten tragen.

3. Mutanten gemäß Anspruch 1 oder 2, **dadurch gekennzeichnet, dass** sie darüber hinaus eine Mutation eines F an Position 383 zu Alanin tragen, und gegebenenfalls eine Mutation von Tryptophan an Position 427 zu Glycin, und/oder von Tryptophan an Position 479 zu Serin.

4. Verwendung von Mutanten gemäß einem der Ansprüche 1 bis 3, als Antigene für die Herstellung von Impfstoffen.
